# NEUE EUROPÄISCHE PATENTSCHRIFT

(11) **EP 1 891 417 B2**
(45) Veröffentlichungstag und Bekanntmachung des Hinweises auf die Entscheidung über den Einspruch: **11.08.2021**
(45) Hinweis auf die Patenterteilung: 23.12.2015
(21) Anmeldenummer: 07711875.0
(22) Anmeldetag: 09.03.2007
(51) Int. Cl.: G01N 21/64, G01N 33/542, G01N 33/58, G01N 35/10, G01N 1/31

(54) **VORRICHTUNG UND VERFAHREN ZUR ERKENNUNG UND IDENTIFIZIERUNG VON ZIELSTRUKTUREN**
DEVICE AND METHOD FOR THE RECOGNITION AND IDENTIFICATION OF TARGET STRUCTURES
DISPOSITIF ET PROCÉDÉ DE DÉTECTION ET D'IDENTIFICATION DE STRUCTURES CIBLES

(30) Priorität: 13.03.2006 DE 102006011467
(43) Veröffentlichungstag der Anmeldung: 27.02.2008
(60) Teilanmeldung: 08161356.4
(73) Patentinhaber: Toposnomos Ltd., 81373 München (DE)
(72) Erfinder: Toposnomos Ltd., 81373 München (DE)
(74) Vertreter: Hofstetter, Schurack & Partner
(86) Internationale Anmeldenummer: PCT/EP2007/002069
(87) Internationale Veröffentlichungsnummer: WO 2007/104486

(56) Entgegenhaltungen:
- EP-A2- 0 810 428
- WO-A-02/18053
- WO-A-96/05488
- WO-A2-02/075280
- US-A- 5 096 670
- US-A1- 2005 153 453
- SCHUBERT WALTER: "TOPOLOGICAL PROTEOMICS, TOPONOMICS, MELK-TECHNOLOGY" ADVANCES IN BIOCHEMICAL ENGINEERING, BIOTECHNOLOGY, SPRINGER, BERLIN, DE, Bd. 83, 2003, Seiten 189-209, XP008079848 ISSN: 0724-6145 in der Anmeldung erwähnt

## Beschreibung

Nach der Entschlüsselung des humanen Genoms besteht die nächste Herausforderung darin, die Protein-Netzwerke einer Zelle zu entschlüsseln, und damit die exakte zelluläre (vs. molekulare) Funktion jedes einzelnen Proteins als Funktion des Kontextes und der Interaktion mit anderen Proteinen zu verstehen. Dazu müssen völlig neue Werkzeuge entwickelt werden. Im Zentrum der zu lösenden technischen Probleme steht die Notwendigkeit Vorrichtungen und Verfahren zu entwickeln, die es ermöglichen eine praktisch beliebige Anzahl verschiedener Proteine oder andere Zielstrukturen in ein und derselben Zelle in nur einem einzigen Experiment zu lokalisieren. Dadurch erst werden die Regeln der räumlichen Assoziationen der Moleküle, Molekülgruppen und insbesondere der Proteine bezogen auf Strukturen erkennbar, so dass in Kombination mit Experimenten (z.B. "Protein Knock Out") oder durch Vergleich verschiedener zellulärer Zustände, wie z.B. in Krankheit und Gesundheit, die exakten zellulären Funktionen dieser Assoziationen entschlüsselt werden können.

Das Problem der Kartierung einer sehr großen Zahl von Molekülen, Molekülgruppen oder insbesondere Proteinen in ein und derselben Zelle ist durch die Entwicklung eines Verfahrens und einer Vorrichtung, wie sie in der EP 0 810 428 B1 beschrieben sind ("Multi-Epitop-Ligand Kartographie" (MELK)) vom Grundsatz her gelöst.

Diese Druckschrift beschreibt eine automatisierte Vorrichtung für die Bestimmung und Messung einer beliebigen Anzahl verschiedener Molekülklassen, Mülekülteile oder Molekülgruppen in flüssigen oder festen Objekten, insbesondere in Zellen und Gewebeproben, welche eine Kombination aus einem Pipettiersystem, einem 3D-Handhabesystem und einem optischen Messsystem, vorzugsweise einen mit einem Bildaufnahmesystem ausgestatteten Mikroskopstativ, umfasst. Ferner beschreibt die Druckschrift ein automatisiertes Verfahren zur Bestimmung und Kartierung einer beliebigen Anzahl von Molekülklassen, Molekülteilen und Molekülgruppen in Objekten, mit dem automatisch eine beliebige Anzahl verschiedener Reagenzlösungen gleichzeitig oder sequentiell auf ein flüssiges oder festes Objekt, insbesondere eine Zelle, Zellen oder eine Gewerbeprobe aufgebracht und gemessen werden kann. Ein wichtiges Anwendungsgebiet stellt die Krebs- und Immunforschung dar. Innerhalb kürzester Zeit werden exakte quantitative und qualitative Auswertungsergebnisse über Anordnung und korrelative Verteilung der Molekülspezies im zu untersuchenden Objekt erhalten, die beispielsweise Aufschluss über Krankheiten und deren Behandlung geben können.

Die Anwendung dieses Verfahrens hat darüber hinaus zu wichtigen neuen Erkenntnissen über die funktionelle Architektur von zellulären Protein-Netzwerken sowie zur Identifikation kombinatorischer Proteinmuster bei Krankheiten geführt (vgl. US 6,638,506, US 6,649,165, US 6,638,515 sowie Schubert W.: "Topological Proteomics, Toponomics, MELK Technology", In: Adv. Biochem. Eng. Biotechnol. 83: 189 - 209 (2003)).

In der Praxis der Anwendung dieses Verfahrens bestehen allerdings noch zahlreiche technische Limitierungen, die den Eintritt dieser Technologie in eine Routineanwendung im Labor, sei es in einem Forschungslabor oder einem Diagnoselabor, erschweren. Dazu zählen folgende Einschränkungen:
1. Durch den in EP 0 810 428 B1 beschriebenen Wechsel von Pipettenspitzen wird relativ viel Zeit auf einen technischen Vorgang verwendet, so dass Zeit für die Markierung von Molekülen, Molekülgruppen oder Proteinen verloren geht. Außerdem ist der Vorgang fortgesetzter Pipettenspitzenwechsel anfällig bezüglich möglicher Ausfälle (Störungen des Pipettenspitzenabwurfs, Störungen der Pipettenspitzenaufnahme etc; nicht korrekt sitzende Pipettenspitze an der Saugeinrichtung des Pipettenspitzen-Aufnahmegerätes mit der Folge des Ansaugens von Luft etc).
2. Die Vorgänge der Markierung von Proteinen und Visualisierung des Markierungsresultates, so wie sie in EP 0 810 428 B1 beschrieben, erfordern pro Einzelmarkierung - bevor ein weiteres Protein markiert werden kann - relativ viel Zeit, so dass derzeit in der Praxis nicht mehr als zwei Proteine pro Zell- oder Gewebeprobe pro Stunde markiert werden können. Diese Tatsachen stehen einer Übersetzung dieser Technologie in einen den Hochdurchsatzverfahren ähnlichen Durchsatzbereich derzeit noch entgegen.

Die US 2005/153453 A1 zeigt den Auftrag einer Spülflüssigkeit auf einen schräg gestellten Teller mit Gewebeproben. Allerdings soll hier der direkte Auftrag auf das Objekt gerade vermieden werden, um die Gewebeproben nicht zu beschädigen. Die Druckschrift befasst sich nicht mit dem Auftrag einer Inkubationsflüssigkeit auf ein biologisches Objekt.

Es besteht die Aufgabe, das in der eingangs genannten EP 0 810 428 B1 beschriebene Verfahren und eine entsprechende Vorrichtung so weiterzubilden, dass eine wesentlich schnellere, automatisierte und kontinuierliche Arbeitsweise ermöglicht wird.

Gelöst wird diese Aufgabe mit den Ansprüchen 1 bzw. 3. Eine vorteilhafte Ausgestaltung ist dem Anspruch 2 entnehmbar.

Die im Folgenden beschriebenen erfindungsgemäßen Vorrichtungen und Verfahren stellen eine Überwindung der genannten Einschränkungen dar.

Automatisiertes Verfahren gemäß EP 0 810 428 B1 zur Durchführung der oben als Stand der Technik angegebenen repetitiven Inkubations-Imaging-Bleaching Zyklen (RIIBZ), mit dem Unterschied, dass in dem automatisierten Prozess kein Pipettenwechsel mehr stattfindet: also alle technischen Parameter in dem Kapitel 0027 des o.g. Patentes sollen beibehalten werden, lediglich Punkt 2 wird dahingehend geändert, dass ein Pipettier-Roboter (3D-Handhabesystem oder ähnlicher Roboter) mit ein- und derselben Pipettenspitze arbeitet, also kein automatisierter Pipettenwechsel mehr stattfindet. Zwischen den einzelnen RIIBZ wird die Pipettenspitze mehrfach in einer Waschlösung gespült, um ggf. vorhandene Reste vorheriger Lösungen zu entfernen.

### Variante A:

An dem Gerät befindet sich eine fest installierte Aufnahme Spitze für das "Andocken" (Aufsetzen) einer Pipettenspitze für die Pipettierung. Diese Pipettenspitze wird manuell vor dem Start des "Toponome Mapping System" bzw. "Toponome Mapping Verfahrens" (TMS) auf die fest installierte Aufnahmespitze aufgesetzt und anschließend wieder manuell entnommen. Zwischendurch wird kein Pipetten-Spitzen-Wechsel durchgeführt. Die Pipettenspitze wird je Einzelzyklus oder auch erst nach mehreren Zyklen des TMS Verfahrens von dem Geräte in Puffer gespült, um dann die nächsten Schritte des TMS Verfahrens durchzuführen.

### Variante B:

An dem Gerät befindet sich eine fest installierte Aufnahme Spitze für das "Andocken" (Aufsetzen) einer Pipettenspitze für die Pipettierung. Diese Pipettenspitze wird automatisch vor dem Start des TMS von dem Pipettiergerät aus einem Container aufgenommen, dann für den gesamten TMS Prozess verwendet und anschließend wieder automatisch, d.h. ohne manuelle Hilfe, wieder abgesetzt. Zwischendurch wird kein Pipetten-Spitzen-Wechsel durchgeführt. Die Pipettenspitze wird je Einzelzyklus oder auch erst nach mehreren Zyklen von dem Gerät in Puffer gespült, um dann die nächsten Schritte des TMS-Verfahrens durchzuführen.

### Variante C:

An dem TMS Gerät befindet sich ein fest installiertes Pipettiergerät, das eine fest installierte Pipettenspitze enthält, welche die verschiedenen Flüssigkeiten automatisch pipettiert. Zwischendurch wird kein Pipetten-Spitzen-Wechsel durchgeführt. Spülungen der Pipettenspitze erfolgen je nach Ablaufprotokoll während und/oder vor bzw. nach den Zyklen des TMS Prozesses.

Alle unter A bis C geschilderten Varianten werden im Unterschied zu dem Verfahren in EP 0810 428 B1 im sog. Shear Flow Pipettiermodus eingesetzt. Dieser besteht darin, dass ohne jeden Pipettenspitzenwechsel mit ein und derselben Pipettenspitze die Inkubation eines jeden einzelnen Tags (Antikörper, Aptamer, Lectin etc.) rasch wiederholt wird, und zwar in der folgenden Weise:

Die von der Pipettenspitze aufgenommene Inkubationsflüssigkeit, die ein bestimmtes Tag enthält, wird auf die biologische Probe (auf dem Inkubationstisch des TMS) schräg aufgespritzt. Es entsteht auf der Probe ein Shear Flow der entsprechenden Inkubationslösung. Dieser führt zu einer verbesserten Tag Reaktion mit dem zu markierenden korrespondierenden Molekül in der Probe (Protein etc). Nach einer variabel programmierbaren, dann folgenden Inkubationszeit (z.B. 1 min) wird dieselbe Inkubationslösung rasch von derselben Pipettenspitze wieder abgesaugt, und anschließend in der beschriebenen schrägen Weise wieder auf dieselbe Probe gespritzt, usw. Dadurch entstehen N Zyklen der Inkubation mit ein und demselben Tag. Nach einer variabel zu bestimmenden Zeit wird dann mit einer Waschlösung gespült, gefolgt von dem Imaging-Vorgang, der die Lokalisation der Tag Reaktion in der Probe registriert.

Vorteil: Im Unterschied zu der klassischen "ruhenden" Dauerinkubation im Tropfen gemäß EP 0810 428 B1 wird die Effizienz der Markierungskinetik gesteigert, so dass der gesamte Inkubationsvorgang zeitlich stark verkürzt wird.

Alle beschriebenen Verfahren werden entweder direkt auf dem Objekttisch im Strahlengang des Mikroskops oder extern (d.h. außerhalb des Strahlengangs des Mikroskops) durchgeführt (entweder auf dem Objekttisch des Mikroskops oder einem gesonderten Inkubationstisch des TMS Gerätes). Im Falle dieser sog. externen Inkubationsprozesse erfolgen die Imaging-Vorgänge immer erst nach positionsgenauer Rückführung des bzw. der zu untersuchenden Gesichtsfelder in den Strahlengang des Mikroskops. Im Falle der Verwendung von Fluorochromen, die an die jeweiligen Tags gekoppelt sind, nun im Falle der Verwendung von Bleichungsvorgängen, erfolgen die Bleichungsvorgänge entweder direkt im Strahlengang des Mikroskops oder extern in einer speziell installierten Bleichungsstation.

## Patentansprüche

1. Verfahren zur Bestimmung und Messung einer beliebigen Anzahl Xn (n = 1,2,3...N) von Zielstrukturen bestehend aus Molekülklassen, Molekülgruppen und Molekülteilen auf einem festen biologischen Objekt bzw. in einem festen biologischen Objekt mittels repetitiver Inkubations-Imaging-Bleaching Zyklen,
**dadurch gekennzeichnet, dass**
- eine automatische Pipettierung für die Aufnahme und Abgabe von mindestens einem Tag enthaltenden Flüssigkeiten unter schrägem Auftrag auf das feste Objekt bzw. in das feste Objekt ohne Pipettenwechsel erfolgt;
- dass zum schrägen Auftrag die von der Pipettenspitze aufgenommene Flüssigkeit auf das biologische Objekt schräg aufgespritzt wird, so dass auf dem Objekt ein Shear Flow der Inkubationslösung entsteht;
- dass Zyklen der Inkubation mit ein und demselben Tag durchgeführt werden, indem mehrfach nach einer variablen Inkubationszeit dieselbe Inkubationslösung rasch von derselben Pipettenspitze wieder abgesaugt und anschließend schräg wieder auf dasselbe Objekt gespritzt wird; und
- dass nach einer variabel zu bestimmenden Zeit dann mit einer Waschlösung gespült wird, gefolgt von einem Imaging-Vorgang, der die Lokalisation der Tag-Reaktion im Objekt registriert.

2. Verfahren nach Anspruch 1,
**dadurch gekennzeichnet, dass**
ein Spülung der Pipette nach einem oder mehreren Inkubations-Imaging-Bleaching Zyklen erfolgt.

3. Automatisierte Vorrichtung zur Bestimmung und Messung einer beliebigen Anzahl Xn (n = 1,2,3...N) von Zielstrukturen bestehend aus Molekülklassen, Molekülgruppen und Molekülteilen auf einem festen biologischen Objekt bzw. in einem festen biologischen Objekt, zur Durchführung des Verfahrens nach Anspruch 1, wobei die Vorrichtung eine Kombination aus einem Pipettiersystem, einem 3D-Handhabesystem und einem optischen Messsystem umfasst, wobei das Pipettiersystem, das 3D-Handhabesystem und das optische Messsystem durch einen Computer gesteuert und kontrolliert werden und das Pipettiersystem eine automatische Pipettierung für die Aufnahme und Abgabe von Flüssigkeiten und eine angemessene Wiederholpositioniergenauigkeit, bevorzugt von mindestens +/- 0,1 mm, sowie eine manuell oder automatisch vor und nach dem Einsatz der Vorrichtung aufsetzbare und abnehmbare oder fest installierte Pipettenspitze aufweist,
**dadurch gekennzeichnet, dass**
die Vorrichtung ausgebildet ist
- für einen schrägen Auftrag der mindestens ein Tag enthaltenden Flüssigkeit auf die Probe die von der Pipettenspitze aufgenommene Flüssigkeit auf das biologische Objekt schräg aufzuspritzen, so dass auf dem Objekt ein Shear Flow der Inkubationslösung entsteht;
- Zyklen der Inkubation mit ein und demselben Tag durchzuführen, indem mehrfach nach einer variablen Inkubationszeit dieselbe Inkubationslösung rasch von derselben Pipettenspitze wieder abgesaugt und anschließend schräg wieder auf dasselbe Objekt gespritzt wird; und
- nach einer variabel zu bestimmenden Zeit dann mit einer Waschlösung zu spülen, gefolgt von einem Imaging-Vorgang, der die Lokalisation der Tag-Reaktion im Objekt registriert.

## Claims

1. A method for determining and measuring any number Xn (n = 1, 2, 3 ... N) of target structures consisting of molecular classes, molecular groups and molecular parts on a solid biological object or in a solid biological object by means of repetitive incubation-imaging-bleaching cycles,
**characterized in that**
- automatic pipetting for receiving and discharging liquids containing at least one tag is effected with oblique application to the solid object or into the solid object without pipette change;
- the liquid received by the pipette tip is obliquely sprayed onto the biological object for oblique application such that a shear flow of the incubation solution arises on the object;
- cycles of the incubation are performed with one and the same tag **in that** the same incubation solution is fast again sucked by the same pipette tip and is subsequently again obliquely sprayed onto the same object multiple times after a variable incubation time; and
- after a time to be variably determined, it is then purged with a wash solution, followed by an imaging procedure, which registers the localization of the tag reaction in the object.

2. The method according to claim 1,
**characterized in that**
purge of the pipette is effected after one or multiple incubation-imaging-bleaching cycles.

3. An automated device for determining and measuring any number Xn (n = 1, 2, 3 ... N) of target structures consisting of molecular classes, molecular groups and molecular parts on a solid biological object or in a solid biological object for performing the method according to claim 1, wherein the device includes a combination of a pipetting system, a 3D handling system and an optical measuring system, wherein the pipetting system, the 3D handling system and the optical measuring system are controlled and monitored by a computer and the pipetting system has automatic pipetting for receiving and discharging liquids and an adequate repetition positioning accuracy, preferably of at least +/- 0.1 mm, as well as a pipette tip capable of being manually or automatically fitted and removed before and after the employment of the device or fixedly installed,
**characterized in that**
the device is formed
- to obliquely spray the liquid received by the pipette tip onto the biological object for an oblique application of the liquid containing at least one tag to the sample, such that a shear flow of the incubation solution arises on the object;
- to perform cycles of the incubation with one and the same tag **in that** the same incubation solution is fast again sucked by the same pipette tip and subsequently again obliquely sprayed onto the same object multiple times after a variable incubation time; and
- to then purge with a wash solution after a time to be variably determined, followed by an imaging procedure, which registers the localization of the tag reaction in the object.

## Revendications

1. Procédé servant à déterminer et à mesurer un nombre quelconque Xn (n = 1, 2, 3, ... N) de structures cibles, constituées de classes de molécules, de groupes de molécules et de parties de molécules sur un objet biologique solide et / ou dans un objet biologique solide au moyen de cycles répétitifs d'incubation-imagerie-blanchiment,
**caractérisé en ce que**
- un pipetage automatique est effectué en vue de la réception et de la distribution de liquides contentant au moins une étiquette par application inclinée sur l'objet solide et / ou dans l'objet solide sans changement de pipette;
- que pour l'application inclinée, le liquide reçu par la pointe de pipette est appliqué de manière inclinée sur l'objet biologique, de sorte qu'un écoulement de cisaillement de la solution d'incubation est créé sur l'objet;
- que des cycles d'incubation sont effectués avec un seul et même étiquette en aspirant à nouveau rapidement et plusieurs fois la même solution d'incubation à partir de la même pointe de pipette après un temps d'incubation variable, et ensuite est appliquée de manière inclinée à nouveau sur le même objet; et
- qu'ensuite, après une période de temps variable, on rince avec une solution de lavage, suivi par un procédé d'imagerie enregistrant la localisation de la réaction de l'étiquette dans l'objet.

2. Procédé selon la revendication 1,
**caractérisé en ce que**
un rinçage de la pipette est effectué après un ou plusieurs cycles d'incubation-imagerie-blanchiment.

3. Dispositif automatisé servant à déterminer et à mesurer un nombre quelconque Xn (n = 1, 2, 3... N) de structures cibles, constituées de classes de molécules, de groupes de molécules et de parties de molécules sur un objet biologique solide et/ou dans un objet biologique solide, servant à la mise en œuvre du procédé selon la revendication 1, dans lequel le dispositif comprend une combinaison composée d'un système de pipetage, d'un système de manutention 3D et d'un système de mesure optique, dans lequel que le système de pipetage, le système de manutention 3D et le système de mesure optique sont commandés et contrôlés par un ordinateur, et le système de pipetage présente un pipetage automatique en vue de la réception et de la distribution de liquides et une précision de repositionnement appropriée, de préférence, d'au moins +/- 0,1 mm, ainsi qu'une pointe de pipette pouvant être montée et retirée manuellement ou automatiquement, avant et après l'utilisation du dispositif, ou installée en permanence,
**caractérisé en ce que**
le dispositif est réalisé,
- pour une application inclinée du liquide contenant au moins une étiquette sur l'échantillon, d'appliquer de manière inclinée le liquide reçu par la pointe de pipette sur l'objet biologique, de sorte qu'un écoulement de cisaillement de la solution d'incubation est créé sur l'objet;
- pour effectuer des cycles d'incubation avec une seul et même étiquette en aspirant à nouveau rapidement et plusieurs fois la même solution d'incubation à partir de la même pointe de pipette après un temps d'incubation variable, et ensuite l'appliquant de manière inclinée à nouveau sur le même objet; et
- pour rincer ensuite, après une période de temps variable, avec une solution de lavage, suivi par un procédé d'imagerie enregistrant la localisation de la réaction de l'étiquette dans l'objet.
